# EUROPÄISCHE PATENTSCHRIFT

(11) **EP 3 248 002 B1**
(45) Veröffentlichungstag und Bekanntmachung des Hinweises auf die Patenterteilung: **23.12.2020**
(21) Anmeldenummer: 16700524.8
(22) Anmeldetag: 12.01.2016
(51) Int. Cl.: G01N 33/497, G01N 21/35

(54) **VERFAHREN ZUM NACHWEIS VON HELICOBACTER PYLORI**
METHOD FOR DETECTING HELICOBACTER PYLORI
PROCÉDÉ DE DÉTECTION D'HELICOBACTER PYLORI

(30) Priorität: 22.01.2015 DE 102015000626
(43) Veröffentlichungstag der Anmeldung: 29.11.2017
(73) Patentinhaber: Kibion GmbH, 28357 Bremen (DE)
(72) Erfinder: WAGNER, Grischa, 28201 Bremen (DE); SILVESTRIC-SCHEEL, Marko, 28209 Bremen (DE)
(74) Vertreter: Möller, Friedrich
(86) Internationale Anmeldenummer: PCT/EP2016/000035
(87) Internationale Veröffentlichungsnummer: WO 2016/116257

(56) Entgegenhaltungen:
- WO-A1-98/27416
- WO-A2-01/75439
- KATHARINA WÖRLE ET AL: "Breath Analysis with Broadly Tunable Quantum Cascade Lasers", ANALYTICAL CHEMISTRY, Bd. 85, Nr. 5, 5. März 2013 (2013-03-05), Seiten 2697-2702, XP055258070, US ISSN: 0003-2700, DOI: 10.1021/ac3030703
- M. B. ESLER ET AL: "Precision Trace Gas Analysis by FT-IR Spectroscopy. 2. The 13 C/ 12 C Isotope Ratio of CO 2", ANALYTICAL CHEMISTRY, Bd. 72, Nr. 1, 1. Januar 2000 (2000-01-01) , Seiten 216-221, XP055257388, US ISSN: 0003-2700, DOI: 10.1021/ac990563x

## Beschreibung

Die Erfindung betrifft ein Verfahren zum Nachweis von Helicobacter Pylori gemäß dem Oberbegriff des Anspruchs 1.

Der ¹³C-Harnstoff-Atemtest hat sich zum Nachweis von Helicobacter Pylori Infektionen, die vielfach Ursache von Erkrankungen des Magen-Darm-Traktes sind, für die klinische Diagnostik etabliert. Bei diesem Atemtest wird der betroffenen Person mit ¹³C markierter Harnstoff verabreicht. Helicobacter Pylori produziert ein Enzym namens Urease, welches den Harnstoff unter anderem in CO₂ zerlegt. Dabei wird das ¹³C-Kohlenstoffisotop des markierten Harnstoffs in das CO₂-Molekül eingebaut. Zum Nachweis einer Infektion mit diesem Bakterium ist somit das Verhältnis zwischen ¹³CO₂ und ¹²CO₂ zu bestimmen, wie beispielsweise in WO 01/75439 A2 beschrieben. Dazu wird der betroffenen Person ein entsprechend markiertes Substrat verabreicht und nach einer gewissen Zeit die Atemluft analysiert. Für die Analyse der Atemluft wird die nichtdispersive Infrarotspektroskopie (NDIR-Spektroskopie) verwendet. Dazu durchstrahlt gefilterte Infrarotstrahlung eine Messkammer, die das zu analysierende Gas enthält. Durch Messung des Absorpstionsspektrums mittels eines Infrarotdetektors lassen sich Aussagen über den ¹³C-/¹²C-Gehalt in dem Gas treffen. Bei einem erhöhten Gehalt von ¹³C ist davon auszugehen, dass die Person mit dem Bakterium Helicobacter Pylori infiziert ist.

Bekannte Verfahren zum Nachweis von Helicobacter Pylori sehen es vor, zunächst eine Messkammer, in welcher der Nachweis erfolgen soll mit einem CO₂-freien Gas zu spülen. Dieses Spülen erfolgt gemäß einer festen Zeit, die üblicherweise 30 s bis 60 s beträgt. Nach dem Spülen der Messkammer wird die gasförmige Atemprobe mit der unter Umständen erhöhten Menge ¹³CO₂ eingelassen. Das Einlassen der Probe in die Messkammer erfolgt nach einer vorgegebenen Zeit von ca. 20 s bis 40 s. Nach dieser Zeit wird das Einlassventil der Messkammer geschlossen und die Probe verteilt sich in der Messkammer homogen. Dieses Homogenisieren ist insbesondere wichtig, um den Anteil des ¹³C-/¹²C-Anteils zuverlässig bestimmen zu können. Die Wartezeit beträgt in der Praxis etwa 40 s bis 60 s. Nach Ablauf dieser Zeit erfolgt die eigentliche Messung an der gasförmigen Probe. Die Messung dauert in der Regel, je nachdem wie viele Messwerte aufgenommen werden sollen, 15 s bis 30 s.

WO98/27416 offenbart ein verwandtes Verfahren zum Nachweis von Helicobacter Pylori.

Insbesondere im klinischen Betrieb muss täglich eine Vielzahl der oben beschriebenen Untersuchung bezüglich einer möglichen Infektion mit Helicobacter Pylori durchgeführt werden. Da das dargestellte Verfahren sehr zeitintensiv ist, gestaltet es sich für die Bewältigung einer großen Anzahl von Untersuchungen als ungeeignet.

Der Erfindung liegt daher die Aufgabe zu Grunde, ein Verfahren zu schaffen, durch das ein schneller Nachweis von Helicobacter Pylori in einer gasförmigen Probe realisierbar ist.

Ein Verfahren zur Lösung dieser Aufgabe weist die Maßnahmen des Anspruchs 1 auf. Demnach ist es erfindungsgemäß vorgesehen, dass die Messung des ¹³C-Gehalts nur so lange durchgeführt wird, bis eine Mindestanzahl von Messwerten des ¹³C-Gehalts einer vorzugebenden Standardabweichung genügt, wobei der ¹³C-Gehalt in der Probe bereits während des Homogenisierens der Probe in der Messkammer gemessen wird. Dies stellt insbesondere einen zeitlichen Vorteil dar. Oftmals ist bereits nach kurzer Zeit die Probe in der Messkammer derart homogenisiert, dass die Messwertaufnahme beginnen kann. Durch dieses Verfahren kann daher erheblich Zeit eingespart werden, da nicht mehr wie bisher pauschal eine bestimmte Zeit gewartet werden muss, bis die Aufnahme der Messwerte durchgeführt werden kann. Vielmehr wird erfindungsgemäß nur gerade solange mit der Aufnahme der Messwerte gewartet wie nötig. Die Standardabweichung, insbesondere die Standardabweichung zu einem Mittelwert, wird dabei vorgegeben bzw. anhand der Messwerte ermittelt. Durch das kontinuierliche bzw. taktweise Messen des ¹³C-Gehalts während des Homogenisierens bzw. während des gesamten Verfahrens, wird es ermöglicht, insbesondere das Homogenisieren der Probe in der

Messkammer zu kontrollieren. Sollten sich bereits beim Homogenisieren Messwerte ergeben, die nur geringfügig von dem Sollwert abweichen, können diese Werte bereits als Ergebniswerte verwendet werden. Dabei ist es erfindungsgemäß vorgesehen, dass stets die letzten Messwerte gespeichert werden, für den Fall, dass diese Werte bereits den Ansprüchen für auswertbare Messwerte genügen.

Insbesondere sieht es die Erfindung vor, dass die ersten aufgenommenen Messwerte des ¹³C-Gehalts in der Probe, insbesondere die ersten zehn, vorzugsweise die ersten zwanzig Messwerte, die der Standardabweichung genügen, als Messergebnisse verwendet werden. Dabei hängt die Anzahl der erforderlichen Messwerte von der vorgegebenen Genauigkeit bzw. Zuverlässigkeit ab. Auch hier kann durch Reduzierung der notwendigen Messwerte die Zeit, die durch das Verfahren in Anspruch genommen wird, reduziert werden.

Ein weiteres Ausfühungsbeispiel der Erfindung sieht es vor, dass sobald die ¹³C-Messwerte der Standardabweichung von höchstens 1 %o, vorzugsweise höchstens 0,2 %o genügen, die ¹³C-Messwerte als Messergebnisse bewertet werden. Dabei sind die Werte für die Standardabweichung variabel und können, je nach Anwendung und Anforderung verändert werden. Somit ist es denkbar, dass die Standardabweichung auch auf 10 %o bzw. 5 ‰ oder gar 0,1 %o festgelegt wird.

Weiter kann es die Erfindung vorsehen, dass das Spülen der Messkammer und das Einlassen der Probe in die Messkammer nur so lange durchgeführt wird, bis ein vorzugebender Schwellenwert des CO₂-Gehalts bzw. des ¹²C-Gehalts in der Messkammer erreicht wird. Dazu wird der CO₂-Gehalt bzw. der ¹²C-Gehalt in der Messkammer während des Spülens der Messkammer und während des Einlassens der Probe in die Messkammer gemessen. Anfangs befindet sich in der Messkammer ein beliebiges Gas. Durch Spülen der Messkammer mit einem CO₂-freien Gas und gleichzeitiges Auspumpen der Kammer durch eine Pumpe wird erreicht, das jegliches Restgas, insbesondere Kohlenstoff bzw. CO₂ aus der Kammer entfernt wird. Während dieses Spülvorgangs wird das Gas kontinuierlich oder taktweise spektroskopiert. Sobald der CO₂-Gehalt in der Messkammer 0,1 % bzw. 0,05 % beträgt, wird das Spülen beendet. Diese Werte können je nach Anforderung geändert werden.

Im nächsten Schritt wird sodann die Probe bzw. die Atemprobe der betroffenen Person in die Messkammer geleitet. Auch während dieses Einleitens der Atemprobe in die Messkammer kann eine Pumpe betrieben werden, um die Atemprobe in die Messkammer hineinzubefördern. Während des Einlassens der Probe erfolgt weiterhin die Messung des CO₂-Gehalts bzw. des CO₂-Volumengehalts. Sobald dieser Anteil mindestens 0,2 % bzw. 0,5 % beträgt, wird das Einlassventil geschlossen, so dass kein weiteres Gas in die Kammer strömen kann. Dieser Volumenanteil hat sich als ausreichend für den Nachweist von ¹³C erwiesen.

Die vorliegende Erfindung sieht es weiter vor, dass der ¹³C-Gehalt und der CO₂- bzw. ¹²C-Gehalt von Sensoren ermittelt werden, die von einer Steuereinheit ausgelesen werden und die Steuereinheit die Messwerte der Sensoren mit den vorzuggebenden Schwellwerten vergleicht. Außerdem lässt sich über diese Steuereinheit der Schwellenwert bzw. die Standardabweichung voreinstellen. Sobald eine Atemprobe der Messkammer zugeführt wird, erfolgt das vorab beschriebene Verfahren automatisch. Es kann jedoch auch vorgesehen sein, dass die einzelnenen Schritte, je nach Ergebnis, einzeln initiiert werden müssen. Weiter kann es die Erfindung vorsehen, dass die Steuereinheit mehrere parallel laufende Messverfahren steuert bzw. mehrere Proben zum Messen des ¹³C-Gehalts koordiniert.

Ein weiteres besonders vorteilhaftes Ausführungsbeispiel der vorliegenden Erfindung sieht es vor, dass Pumpen und/oder Ventile zum Regeln eines GasStroms im bzw. aus der Messkammer durch die Steuereinheit geregelt werden. Somit werden in Abhängigkeit der Messwerte Ventile geschlossen bzw. Pumpen an- oder abgestellt und/oder Messwerte ausgelesen.

Ein bevorzugtes Ausführungsbeispiel des erfindungsgemäßen Verfahrens wird anhand einer folgenden Figur näher erläutert.
- Figur: Zeitlicher Verlauf eines Kohlenstoffanteils in einer Messkammer.

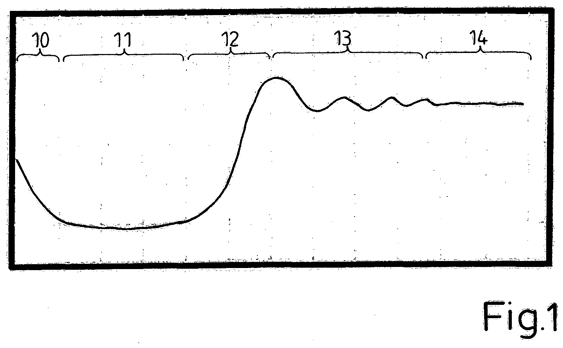

In der Figur ist der zeitliche Verlauf des Kohlenstoff-, insbesondere des ¹³C-Anteils in der Messkammer während des erfindungsgemäßen Verfahrens aufgetragen. Die Ordinate stellt in den willkürlichen Einheiten den relativen Gehalt des Kohlenstoffs in der Messkammer dar.

In einer ersten Phase 10 des Verfahrens ist die Messkammer mit einem beliebigen Gas und somit mit einem beliebigen Gehalt an Kohlenstoff bzw. CO₂ gefüllt. Beispielsweise wurde gerade eine Messung zum Nachweis von Helicobacter Pylori beendet. In der zweiten Phase 11 wird die Messkammer gespült, d.h. für das eigentliche Messverfahren vorbereitet. Dazu wird ein CO₂-freies Gas in die Kammer geleitet und gleichzeitig eine Pumpe, die mit der Kammer verbunden ist, angeschaltet. Durch das Spülen und Pumpen nimmt der Kohlenstoffanteil in der Messkammer rapide ab. Bereits während dieser zweiten Phase 11 des Verfahrnes wird der Kohlenstoffanteil durch NDIR-Spektroskopie bestimmt. Sobald der Kohlenstoffanteil einen bestimmten Schwellenwert unterschritten hat, wird das Spülen beendet, indem der Einlass des CO₂-freien Gases beendet wird.

In der dritten Phase 12 des Verfahrens wir die Atemprobe der betroffenen Person der Messkammer zugeführt. Da der CO₂-Anteil in der Atemprobe hoch ist, steigt der Kohlenstoffanteil in der drittem Phase 12 rapide an. Auch in dieser Phase wird der Kohlenstoffanteil mittels Spektroskopie bestimmt. Sobald der CO₂-, insbesondere der ¹²C- und/oder ¹³C-Anteil einem gewissen Schwellenwert genügt, wird auch der Einlass der Atemprobe gestopt, indem ein Ventil geschlossen wird.

In der folgenden vierten Phase 13 erfolgt eine Homogenisierung der CO₂ aufweisenden Atemprobe in der Messkammer. Während der vierten Phase 13 erfolgt bereits eine gezielte Messung des ¹³C-Anteils (fünfte Phase 14). Somit geht die vierte Phase 13 fließend in die fünfte Phase 14 über. Wenn die ¹³C-Messwerte bereits ergeben, dass die Abweichungen einer voreingestellten Standardabweichung genügen, werden diese Messwerte bereits als Messergebnisse verwertet. Üblicherweise werden 10 bis 20 derartige Messergebnisse bzw. -Punkte aufgenommen, um ein aussagekräftiges Ergebnis bzgl. der Infektion mit Helicobacter Pylori zu erzielen. Nach der fünften Phase 14 kann deas Verfahren gemäß der ersten Phase 10 fortgesetzt werden.

### Bezugszeichenliste

- 10: 1. Phase
- 11: 2. Phase
- 12: 3. Phase
- 13: 4. Phase
- 14: 5. Phase

## Patentansprüche

1. Verfahren zum Nachweis von Helicobacter Pylori mittels Nichtdispersiver-InfrarotSpektroskopie unter Verwendung von ¹³C-markiertem Harnstoff, wobei zunächst eine Messkammer mit CO₂-freiem Gas gespült wird, eine gasförmige Probe in die Messkammer eingelassen wird, die Probe sich homogen verteilt und der ¹³C-Gehalt in der Probe gemessen wird, **dadurch gekennzeichnet, dass** eine Standardabweichung einer Mindestanzahl von mehreren fortlaufenden Messwerten des ¹³C-Gehalts ermittelt wird und die Messung des ¹³C-Gehalts nur solange durchgeführt wird, bis diese ermittelte Standardabweichung einer vorzugebenden Standardabweichung genügt und wobei der ¹³C-Gehalt in der Probe bereits während des Homogenisierens der Probe in der Messkammer gemessen wird.

2. Verfahren nach Anspruch 1, **dadurch gekennzeichnet, dass** die ersten aufgenommenen Messwerte des ¹³C-Gehalts in der Probe, insbesondere die ersten 10, vorzugsweise die ersten 20 Messwerte, die der Standardabweichung genügen, als Messergebnisse verwendet werden.

3. Verfahren nach Anspruch 1 oder 2, **dadurch gekennzeichnet, dass** sobald die ¹³C-Messwerte der Standardabweichung von höchstens 1 %o, vorzugsweise höchstens 0,2 %o, genügen, die ¹³C-Messwerte als Messergebnisse bewertet werden.

4. Verfahren nach einem der vorhergehenden Ansprüche, **dadurch gekennzeichnet, dass** das Spülen der Messkammer und das Einlassen der Probe in die Messkammer nur solange durchgeführt wird, bis ein vorzugebender Schwellenwert des CO₂-Gehalts bzw. des ¹²C-Gehalts in der Messkammer erreicht wird.

5. Verfahren nach einem der vorhergehenden Ansprüche, **dadurch gekennzeichnet, dass** der CO₂-Gehalt bzw. der ¹²C-Gehalt in der Messkammer während des Spülens der Messkammer und während des Einlassens der Probe in die Messkammer gemessen wird.

6. Verfahren nach einem der vorhergehenden Ansprüche, **dadurch gekennzeichnet, dass** die Messkammer solange mit dem CO₂ - freiem Gas gespült wird, bis der CO₂-Gehalt in der Messkammer höchstens 0,1 %, vorzugsweise höchstens 0,05 %, beträgt und darauf die Probe in die Messkammer eingelassen wird.

7. Verfahren nach einem der vorhergehenden Ansprüche, **dadurch gekennzeichnet, dass** die Probe solange in die Messkammer eingelassen wird, bis der CO₂-Volumen-Anteil mindestens 0,2 %, vorzugsweise mindestens 0,5 % beträgt.

8. Verfahren nach einem der vorhergehenden Ansprüche, **dadurch gekennzeichnet, dass** der ¹³C-Gehalt und der CO₂- bzw. ¹²C-Gehalt von Sensoren ermittelt werden, die von einer Steuereinheit ausgelesen werden und die Steuereinheit die Messwerte der Sensoren mit den vorzugebenden Schwellenwerten vergleicht.

9. Verfahren nach einem der vorhergehenden Ansprüche, **dadurch gekennzeichnet, dass** Pumpen und/oder Ventile zum Regeln eines Gasstroms in bzw. aus der Messkammer durch die Steuereinheit geregelt werden.

## Claims

1. A method for detecting Helicobacter pylori by means of non-dispersive infrared spectroscopy with use of ¹³C-labeled urea, wherein firstly a measurement chamber is flushed with CO₂-free gas, a gaseous sample is admitted into the measurement chamber, the sample distributes itself homogeneously and the ¹³C content in the sample is measured, **characterized in that** a standard deviation of a minimum number of a plurality of continuous measurement values of the ¹³C content is determined and the measurement of the ¹³C content is only carried out until this determined standard deviation meets a standard deviation to be specified, and wherein the ¹³C content in the sample is already measured during the homogenization of the sample in the measurement chamber.

2. The method as claimed in claim 1, **characterized in that** the first recorded measurement values of the ¹³C content in the sample, in particular the first 10, preferably the first 20, measurement values which meet the standard deviation are used as measurement results.

3. The method as claimed in claim 1 or 2, **characterized in that** as soon as the ¹³C measurement values meet the standard deviation of at most 1⁰/₀₀, preferably at most 0.2⁰/₀₀, the ¹³C measurement values are assessed as measurement results.

4. The method as claimed in one of the previous claims, **characterized in that** the flushing of the measurement chamber and the admission of the sample into the measurement chamber is performed only until a threshold value of the CO₂ content or the ¹²C content in the measurement chamber, to be specified, is reached.

5. The method as claimed in one of the previous claims, **characterized in that** the CO₂ content or the ¹²C content in the measurement chamber is measured during the flushing of the measurement chamber and during the admission of the sample into the measurement chamber.

6. The method as claimed in one of the previous claims, **characterized in that** the measurement chamber is flushed with the CO₂-free gas until the CO₂ content in the measurement chamber is at most 0.1%, preferably at most 0.05% and thereupon the sample is admitted into the measurement chamber.

7. The method as claimed in one of the previous claims, **characterized in that** the sample is admitted into the measurement chamber until the CO₂ volume content is at least 0.2%, preferably at least 0.5%.

8. The method as claimed in one of the previous claims, **characterized in that** the ¹³C content and the CO₂ content or ¹²C content are determined by sensors which are read off by a control unit and the control unit compares the measurement values of the sensors with the threshold values to be specified.

9. The method as claimed in one of the previous claims, **characterized in that** the pumps and/or valves for regulating a gas flow respectively into and out of the measurement chamber are regulated by the control unit.

## Revendications

1. Procédé de détection d'Helicobacter Pylori par spectroscopie infrarouge non dispersive en utilisant de l'urée marquée par du ¹³C, dans lequel une chambre de mesure est tout d'abord rincée avec un gaz exempt de CO₂, un échantillon gazeux est introduit dans la chambre de mesure, l'échantillon se répartit de manière homogène et la teneur en ¹³C dans l'échantillon est mesurée, **caractérisé en ce qu'**un écart-type d'un nombre minimal de plusieurs valeurs de mesure consécutives de la teneur en ¹³C est déterminé et la mesure de la teneur en ¹³C n'est réalisée que jusqu'à ce que cet écart-type déterminé satisfasse un écart-type prédéterminé, et dans lequel la teneur en ¹³C dans l'échantillon est déjà mesurée pendant l'homogénéisation de l'échantillon dans la chambre de mesure.

2. Procédé selon la revendication 1, **caractérisé en ce que** les premières valeurs mesurées enregistrées de la teneur en ¹³C dans l'échantillon, notamment les 10 premières, de préférence les 20 premières valeurs mesurées, qui satisfont l'écart-type sont utilisées en tant que résultats de mesure.

3. Procédé selon la revendication 1 ou 2, **caractérisé en ce que** dès que les valeurs mesurées de ¹³C satisfont l'écart-type d'au plus 1 ‰, de préférence d'au plus 0,2 ‰, les valeurs mesurées de ¹³C sont évaluées en tant que résultats de mesure.

4. Procédé selon l'une quelconque des revendications précédentes, **caractérisé en ce que** le rinçage de la chambre de mesure et l'introduction de l'échantillon dans la chambre de mesure ne sont réalisés que jusqu'à ce qu'une valeur seuil prédéterminée de la teneur en CO₂ ou de la teneur en ¹²C dans la chambre de mesure soit atteinte.

5. Procédé selon l'une quelconque des revendications précédentes, **caractérisé en ce que** la teneur en CO₂ ou la teneur en ¹²C dans la chambre de mesure est mesurée pendant le rinçage de la chambre de mesure et pendant l'introduction de l'échantillon dans la chambre de mesure.

6. Procédé selon l'une quelconque des revendications précédentes, **caractérisé en ce que** la chambre de mesure est rincée avec le gaz exempt de CO₂ jusqu'à ce que la teneur en CO₂ dans la chambre de mesure soit d'au plus 0,1 %, de préférence d'au plus 0,05 %, et l'échantillon est alors introduit dans la chambre de mesure.

7. Procédé selon l'une quelconque des revendications précédentes, **caractérisé en ce que** l'échantillon est introduit dans la chambre de mesure jusqu'à ce que la proportion volumique de CO₂ soit d'au moins 0,2 %, de préférence d'au moins 0,5 %.

8. Procédé selon l'une quelconque des revendications précédentes, **caractérisé en ce que** la teneur en ¹³C et la teneur en CO₂ ou ¹²C sont déterminées par des capteurs, qui sont lus par une unité de commande, et l'unité de commande compare les valeurs mesurées des capteurs avec les valeurs seuils prédéterminées.

9. Procédé selon l'une quelconque des revendications précédentes, **caractérisé en ce que** des pompes et/ou des soupapes pour la régulation d'un courant gazeux entrant ou sortant de la chambre de mesure sont régulées par l'unité de commande.
